(19) Europäisches Patentamt
European Patent Office
Office européen des brevets

(11) Veröffentlichungsnummer: **0 414 637 A1**

(12) # EUROPÄISCHE PATENTANMELDUNG

(21) Anmeldenummer: **90810609.9**

(51) Int. Cl.[5]: **C07C 317/28**

(22) Anmeldetag: **14.08.90**

(30) Priorität: **23.08.89 CH 3053/89**

(43) Veröffentlichungstag der Anmeldung:
**27.02.91 Patentblatt 91/09**

(84) Benannte Vertragsstaaten:
**CH DE FR GB LI**

(71) Anmelder: **CIBA-GEIGY AG**
**Klybeckstrasse 141**
**CH-4002 Basel(CH)**

(72) Erfinder: **Scherrer, Wilfried**
**Feldstrasse 10,**
**CH-4416 Bubendorf(CH)**
Erfinder: **Studer, Mario, Dr.**
**Sichternstrasse 18**
**CH-4410 Liestal(CH)**

(54) **Verfahren zur Herstellung von aromatischen Aminen.**

(57) Die Erfindung betrifft ein Verfahren zur Herstellung von aromatischen Aminen der Formel

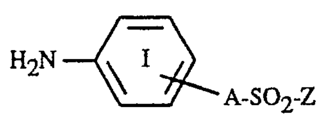

(1),

worin A ein Brückenglied, Z ein Rest der Formel $-CH_2-CH_2-OH$, $-CH=CH_2$ oder $-CH_2-CH_2-Y$, und Y eine unter alkalischen Bedingungen abspaltbare Gruppe ist, und der Benzolkern I weitersubstituiert sein kann, welches dadurch gekennzeichnet ist, dass man Verbindungen der Formel

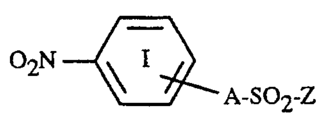

(2),

worin A, Z und I die unter Formel(1)angegebenen Bedeutungen haben, bei einem Druck von 0,1 bis 100 bar in Gegenwart einer niederen aliphatischen oder aromatischen Mono-oder Dicarbonsäure und in Gegenwart eines Nickel-Katalysators hydriert.

Die nach dem erfindungsgemässen Verfahren erhaltenen Verbindungen eignen sich als Zwischenprodukte zur Herstellung von Reaktivfarbstoffen.

EP 0 414 637 A1

## VERFAHREN ZUR HERSTELLUNG VON AROMATISCHEN AMINEN

Die Erfindung betrifft ein neues Verfahren zur Herstellung aromatischer Amine, die einen faserreaktiven Rest des Vinylsulfonyltyps enthalten, aus den entsprechenden aromatischen Nitroverbindungen in Gegenwart von Nickelkatalysatoren und einer niederen aliphatischen oder aromatischen Mono- oder Dicarbonsäure.

In den letzten Jahren ist man zunehmend bestrebt, Herstellungsverfahren für chemische Verbindungen, insbesondere für Farbstoffe und deren Zwischenprodukte zu optimieren, und zwar sowohl was den Herstellungsprozess anbetrifft, als auch hinsichtlich der Aufarbeitung. Um hier zu befriedigenden und reproduzierbaren Ergebnissen zu gelangen, ist man auf Herstellungsverfahren angewiesen, die sich durch folgende Kriterien auszeichnen: vollständige Umsetzung, geringer Nebenproduktanteil, einfache Verfahrensführung, Automationsfähigkeit, kurze Verweilzeiten in den Reaktionsgefässen und einfachste Aufarbeitungs- bzw. Abtrennmethode.

Zur Herstellung aromatischer Amine aus den entsprechenden Nitroverbindungen sind aus der Literatur zahlreiche Verfahren bekannt. Weniger bekannt sind Herstellungsverfahren aronatischer Amine aus den entsprechenden Nitroverbindungen, die einen faserreaktiven Rest enthalten. Nachteil der meisten dieser Reduktionen bzw. Hydrierungsreaktionen ist sowohl die unbefriedigende Ausbeute als auch der erhebliche Zeitaufwand für diese Umsetzung. Ferner sind Reduktionsreaktionen bekannt, die bedingt durch grössere Salzmengen spezielle Aufarbeitungen erforderlich machen.

Ueberraschenderweise erlaubt das erfindungsgemässe Verfahren die Herstellung aromatischer Amine, die einen faserreaktiven Rest enthalten, aus den entsprechenden Nitroverbindungen auf einfache Art und Weise, ohne die genannten Nachteile aufzuweisen.

Gegenstand der Erfindung ist ein Verfahren zur Herstellung von aromatischen Aminen der Formel

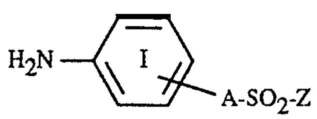

(1),

worin A ein Brückenglied, Z ein Rest der Fomel $-CH_2-CH_2-OH$, $-CH=CH_2$ oder $-CH_2-CH_2-Y$, und Y eine unter alkalischen Bedingungen abspaltbare Gruppe ist, und der Benzolkern I weitersubstituiert sein kann, welches dadurch gekennzeichnet ist, dass man Verbindungen der Formel

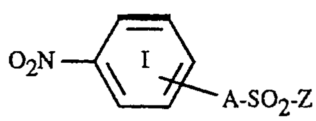

(2),

worin A, Z und I die unter Formel (1) angegebenen Bedeutungen haben, bei einem Druck von 0,1 bis 100 bar in Gegenwart einer niederen aliphatischen oder aromatischen Mono-oder Dicarbonsäure und in Gegenwart eines Nickel-Katalysators hydriert.

Bei diesem Vorgehen erhält man überraschenderweise aromatische Amine der Formel (1) in nahezu quantitativer Ausbeute und nach einer Reaktionszeit von nur 1 bis 2 Stunden.

Die in dem erfindungsgemässen Verfahren verwendeten aromatischen Nitroverbindungen der Formel (2) können im Benzolkern weitersubstituiert sein. Als Substituenten des Benzolkerns kommen z.B. die folgenden in Betracht:

$C_1$-$C_4$-Alkyl, wie Methyl, Äthyl, Propyl, Isopropyl, Butyl, Isobutyl, sek.-Butyl und tert.-Butyl, $C_1$-$C_4$-Alkoxy, wie Methoxy, Äthoxy, Propoxy, Isopropoxy, sek.-Butoxy, tert.-Butoxy, Isobutoxy und n-Butoxy, Halogen, wie Fluor, Chlor und Brom und Sulfo. Bevorzugt sind die Substituenten Methyl, Äthyl, Methoxy, Äthoxy, Chlor, Brom und Sulfo.

Als Brückenglied A kommen z.B. die folgenden Reste in Betracht:

$$-CON(R_3)-(CH_2)_m- \;,\; -(O)_{0-1}CH_2-CON(R_3)-(CH_2)_m- \text{ oder } -N(R_3)- \text{ wie z.B.}$$

$-CONHCH_2CH_2-$, $-CH_2CONHCH_2CH_2-$, $-OCH_2CONHCH_2CH_2-$ und -N(H)-, wobei $R_3$ Wasserstoff, Methyl oder Aethyl bedeutet und m = 2,3,4,5 oder 6 ist. Der Rest $-SO_2Z$ ist an die $CH_2$-Gruppe der genannten

Reste bzw. an das Stickstoffatom im Rest -N($R_3$)- gebunden.

Bei Y handelt es sich z.B. um einen unter alkalischen Bedingungen abspaltbaren anorganischen oder organischen Rest.

Beispiele für geeignete Reste Y sind:
-$OSO_3H$, -$SSO_3H$, -$OCOCH_3$, -OCO-$C_6H_5$, $OPO_3H_2$, -Cl, -Br, -F,

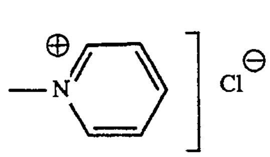 , 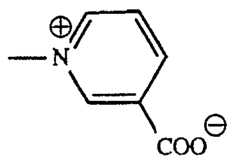 , 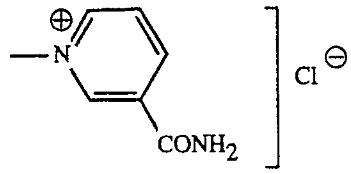 .

Vorzugsweise steht Y für die Gruppe -$OSO_3H$, -$OCOCH_3$, -$OPO_3H_2$ oder Cl und besonders bevorzugt für die Gruppe -$OSO_3H$.

Als niedere aliphatische oder aromatische Mono- oder Dicarbonsäuren kommen z.B. solche der Formeln
$R_1COOH$ oder $R_2(COOH)_2$
in Betracht, worin $R_1$ lineares oder verzweigtes $C_1$-$C_6$-Alkyl, Cyclohexyl, Phenyl oder Benzyl ist, und $R_2$ eine direkte Bindung oder lineares oder verzweigtes $C_2$-$C_6$-Alkylen, Cyclohexylen oder Phenylen darstellt. Als Beispiele seien genannt: Essigsäure, Propionsäure, Buttersäure, Isobuttersäure, Malonsäure, Bernsteinsäure, Oxalsäure, Maleinsäure, Fumarsäure und Benzoesäure.

Bevorzugt werden in dem erfindungsgemässen Verfahren aliphatische Mono- oder Dicarbonsäuren mit 2 bis 4 Kohlenstoffatomen verwendet, sowie Benzoesäure als aromatische Monocarbonsäure.

Eine besonders bevorzugte Ausführungsform des erfindungsgemässen Verfahrens ist dadurch gekennzeichnet, dass man Essigsäure verwendet.

In dem erfindungsgemässen Verfahren werden die niederen aliphatischen oder aromatischen Mono- oder Dicarbonsäuren vorzugsweise in einer Menge von 0,1 bis 10 Molprozent, insbesondere 3 bis 5 Molprozent, bezogen auf die molare Menge der Verbindung der Formel (2) verwendet.

Die Hydrierung der Verbindung der Formel (2) erfolgt vorzugsweise in Gegenwart von 0,5 bis 20 Gewichtsprozent, insbesondere in Gegenwart von 1 bis 10 Gewichtsprozent Nickel-Katalysator bezogen auf die Menge der Verbindung der Formel (2).

Als Nickel-Katalysatoren haben sich Nickel-Trägerkatalysatoren, insbesondere Nickel auf Kieselgur, oder Raney-Nickel in dem erfindungsgemässen Verfahren bewährt.

Bevorzugte Ausführungsformen des erfindungsgemässen Verfahrens sind dadurch gekennzeichnet, dass man

a) bei einer Temperatur von 30 bis 150 °C, insbesondere bei einer Temperatur zwischen 50 und 140 °C hydriert; und/oder

b) bei einem pH-Wert von 4,5 bis 7, insbesondere bei einem pH-Wert von 5 bis 7, hydriert; und/oder

c) vorzugsweise bei einem Druck von 1 bis 40 bar hydriert.

Die Hydrierung kann z.B. in Substanz oder in einem inerten Lösungsmittel durchgeführt werden. Geeignete Lösungsmittel sind z.B. Alkanole (Methanol, Ethanol, Propanol, Butanol, Methoxy- oder Ethoxyethanol), Ether (Dibutylether, t-Butylmethylether, Tetrahydrofuran, Dioxan, Ethylenglykoldimethylether, Diethylenglykoldimethylether), Amide und Lactame (Dimethylformamid, Dimethylacetamid, N-Methylpyrrolidon), Ester und Lactone (Essigsäureethylester, $\gamma$-Butyrolacton), Kohlenwasserstoffe (Pentan, Hexan, Methylcyclohexan, Benzol, Toluol, Xylol, Chlorbenzol), Wasser. Bei der Hydrierung in Substanz ist das bei der Hydrierung entstehende Amin das Lösungsmittel.

In einer bevorzugten Ausführungsform wird als Lösungsmittel Wasser verwendet.

Die Reaktionszeit richtet sich im wesentlichen nach den Reaktionsbedingungen und beträgt im allgemeinen weniger als zwei Stunden.

Das erfindungsgemässe Verfahren kann so durchgeführt werden, dass man in einem Autoklaven die Nitroverbindung, den Nickelkatalysator, das Lösungsmittel und die Mono- oder Dicarbonsäure einträgt und die Luft zuerst durch Stickstoff und diesen dann durch Wasserstoff verdrängt. Dann wird der Autoklav verschlossen, Wasserstoff bis zum gewünschten Druck aufgepresst und auf die Reaktionstemperatur erhitzt. Nach Beendigung der Reaktion wird das Reaktionsgemisch vom Katalysator abgetrennt. Danach kann man das Reaktionsprodukt vom Reaktionswasser und Lösungsmittel abtrennen und durch Destillation oder Umkristallisation weiter reinigen. Die primären aromatischen Amine sind bekannter weise Zwischenprodukte zur Herstellung von Farbstoffen.

Eine besonders bevorzugte Ausführungsform des erfindungsgemässen Verfahrens ist dadurch gekennzeichnet, dass man Verbindungen der Formel

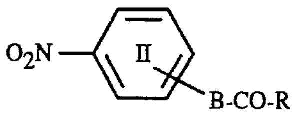 (2a)

verwendet, worin B die direkte Bindung oder ein Rest der Formel

$$-(CH_2)_n- \text{ oder } -O-(CH_2)_n- ,$$

n = 1 bis 6, R ein Rest der Formel

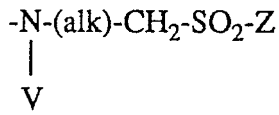 (3a),

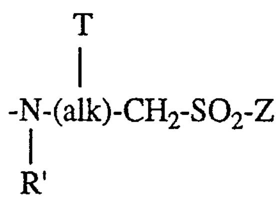 (3b),

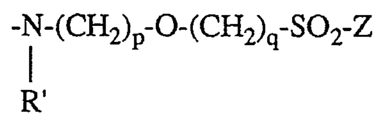 (3c),

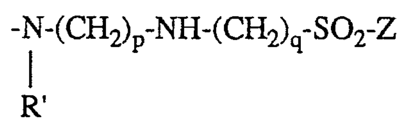 (3d),

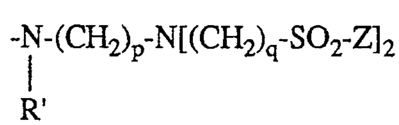 (3e) oder

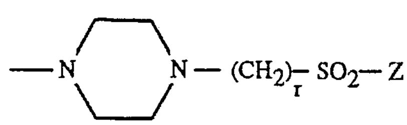 (3f),

worin R′ Wasserstoff oder $C_1$-$C_6$-Alkyl ist, (alk) einen $C_1$-$C_6$-Alkylenrest darstellt, T Wasserstoff, Halogen, Hydroxy, Sulfato, Carboxy, Cyano, $C_1$-$C_4$-Alkoxycarbonyl, Carbamoyl oder einen Rest $-SO_2$-Z bedeutet, V Wasserstoff, gegebenenfalls substituiertes $C_1$-$C_4$-Alkyl oder einen Rest der Formel -(alk)-$CH_2$-$SO_2$-Z, worin (alk) die zuvor angegebene Bedeutung hat, ist, Z die unter Formel (1) angegebene Bedeutung hat, und p, q und r unabhängig voneinander eine ganze Zahl von 1 bis 6 bedeuten, ist, und der Benzolkern II weitere

Substituenten enthalten kann.

Bei R′ in den Formeln (3b) bis (3e) handelt es sich z.B. um Wasserstoff, Methyl, Ethyl, n-oder iso-Propyl, n-, sec.- oder tert.-Butyl oder um einen geradkettigen oder verzweigten Pentyl- oder Hexylrest. Vorzugsweise steht R′ für Wasserstoff, Methyl oder Ethyl und besonders bevorzugt für Wasserstoff.

(alk) wie z.B. in den Formeln (3a) und (3b) bedeutet z.B. einen Methylen-, Ethylen, 1,3-Propylen-, 1,4-Butylen-, 1,5-Pentylen- oder 1,6-Hexylenrest oder dessen verzweigte Isomere. Bevorzugt ist für (alk) die Bedeutung eines $C_1$-$C_4$-Alkylenrestes und insbesondere die Bedeutung Methylen oder Ethylen.

p und q in den Formeln (3c), (3d) und (3e) stehen unabhängig voneinander bevorzugt für eine ganze Zahl von 1 bis 4; besonders bevorzugt bedeuten p und q jeweils die Zahl 2.

T steht vorzugsweise für Wasserstoff, Hydroxy, Sulfato, Acetyloxy, Carboxy, Methoxycarbonyl, Ethoxycarbonyl oder die Gruppe $-SO_2$-Z, wobei Z die zuvor angegebene Bedeutung hat; besonders bevorzugt ist T Wasserstoff oder ein Rest $-SO_2$-Z.

Ist V ein substituierter $C_1$-$C_4$-Alkylrest, so kann dies z.B. durch Halogen, Hydroxy, Cyano, Carboxy, Sulfo, Sulfato, $C_1$-$C_4$-Alkoxy oder $C_1$-$C_4$-Alkoxycarbonyl substituierter $C_1$-$C_4$-Alkylrest sein.

Beispiele für substituierte $C_1$-$C_4$-Alkylreste sind: Carboxymethyl, β-Carboxyethyl, Methoxycarbonylmethyl, Ethoxycarbonylmethyl, β-Carboxyethyl, Methoxycarbonylmethyl, Ethoxycarbonylmethyl, β-Methoxyethyl, β-Ethoxyethyl, β-Chlorethyl, γ-Brompropyl, β-Hydroxyethyl, β-Hydroxybutyl, β-Cyanethyl, Sulfomethyl, β-Sulfoethyl, β-Sulfatoethyl.

Steht V für einen Rest der Formel $Z-O_2S-H_2C$-(alk)-, so kann sich dieser von dem in Formel (3a) vorhandenen zweiten Rest $Z-O_2S-H_2C$-(alk)- unterscheiden oder, vorzugsweise, mit letzterem übereinstimmen.

V bedeutet vorzugsweise Wasserstoff, Methyl, Ethyl oder die Gruppe $Z-O_2S-H_2C$(alk)-; insbesondere bevorzugt ist für V die Bedeutung Wasserstoff.

Beispiele für besonders bevorzugte Reste R sind:

$-NH-(CH)_2-SO_2-(CH_2)_2-OSO_3H$

$-NH-(CH_2)_3-SO_2-(CH_2)_2-OSO_3H$

$-NH-(CH_2)_2-O-(CH_2)_2-SO_2-(CH_2)_2-OSO_3H$

$-N[-(CH_2)_2-SO_2-(CH_2)_2-OSO_3H]_2$

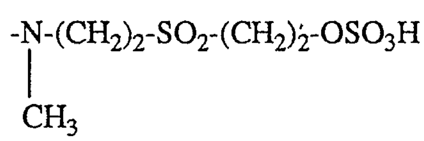

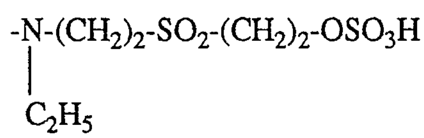

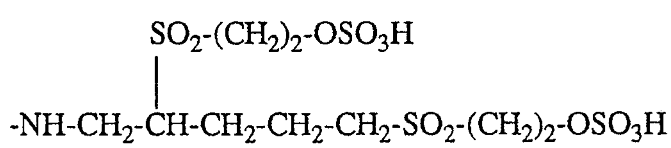

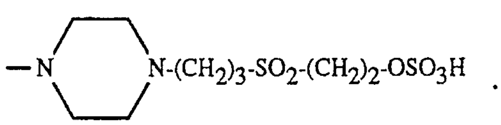

sowie die entsprechenden Reste, die anstelle der Gruppe -O $SO_3H$ die Gruppe -OH enthalten.

In dem erfindungsgemässen Verfahren geht man insbesondere von Verbindungen der Formel (2) bzw. (2a) aus, deren Benzolkern ausser der $O_2N$-Gruppe und dem Rest $-A-SO_2-Z$ keine weiteren Substituenten enthält.

Eine ganz besonders bevorzugte Ausführungsform des erfindungsgemässen Verfahrens ist dadurch gekennzeichnet, dass man Verbindungen der Formel (2) oder (2a) verwendet, worin Z β-Hydroxyäthyl, β-Sulfatoäthyl, β-Thiosulfatoäthyl, β-Phosphatoäthyl, β-Acetoxyäthyl, β-Halogenäthyl oder Vinyl ist.

Eine ganz besonders bevorzugte Ausführungsform des erfindungsgemässen Verfahrens zur Herstellung von Verbindungen der Formel

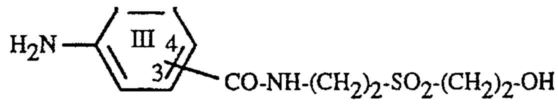

(4)

wobei der Rest $CO-NH-(CH_2)_2-SO_2-(CH_2)_2-OH$ in 3- oder 4-Stellung an den Benzring III gebunden ist, ist dadurch gekennzeichnet, dass man Verbindungen der Formel

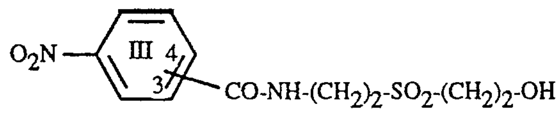

(5)

wobei der Rest $CO-NH-(CH_2)_2-SO_2-(CH_2)_2-OH$ in 3- oder 4-Stellung an den Benzring III gebunden ist, bei einem Druck von 15 bis 25 bar, insbesondere 20 bar, bei einer Temperatur von 70 bis 90 °C, insbesondere 80 °C, in Gegenwart von 2 bis 4 Molprozent Raney-Nickel bezogen auf die Verbindung der Formel (5), insbesondere in Gegenwart von 3 Molprozent Raney-Nickel bezogen auf die Verbindung der Formel (5) bei einem pH-Wert von 5 bis 5,5 in Gegenwart von Essigsäure hydriert.

Eine ebenfalls ganz besonders bevorzugte Ausführungsform des erfindungsgemässen Verfahrens zur Herstellung von Verbindungen der Formel

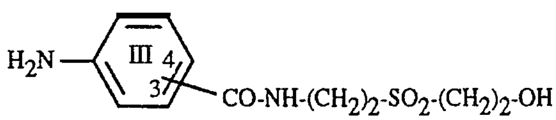

(4)

wobei der Rest $CO-NH-(CH_2)_2-SO_2-(CH_2)_2-OH$ in 3- oder 4-Stellung an den Benzring III gebunden ist, ist dadurch gekennzeichnet, dass man Verbindungen der Formel

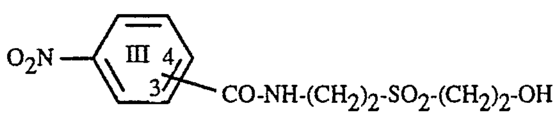

(5)

wobei der Rest $CO-NH-(CH_2)_2-SO_2-(CH_2)_2-OH$ in 3- oder 4-Stellung an den Benzring III gebunden ist, bei einem Druck von 15 bis 25 bar, insbesondere 20 bar, bei einer Temperatur von 80 bis 120 °C, insbesondere 100 bis 120 °C, in Gegenwart von 3 bis 6 Molprozent eines Nickel-Kieselgur-Trägerkatalysators (mit einem Nickelgehalt von 55 Gewichtsprozent) bezogen auf die Verbindung der Formel (5), insbesondere in Gegenwart von 4 bis 5 Molprozent eines Nickel-Kieselgur-Trägerkatalysators (mit einem Nickelgehalt von 55 Gewichtsprozent), bezogen auf die Verbindung der Formel (5) bei einem pH-Wert von 6 bis 7 in Gegenwart von Essigsäure hydriert.

Die erfindungsgemäss erhaltenen Verbindungen der Formel (1), worin Z $\beta$-Hydroxyäthyl ist, können in andere Verbindungen der Formel (1) umgewandelt werden, d.h. im Anschluss an die Hydrierung der Verbindung der Formel (2), worin Z $\beta$-Hydroxyäthyl ist, erfolgt gegebenenfalls eine Umwandlungsreaktion, z.B. eine Veresterung oder Dehydratisierung.

So können Verbindungen der Formel (1), in denen die Gruppierung $-SO_2-Z$ eine $\beta$-Hydroxyäthylsulfonylgruppe darstellt, durch Behandeln mit Sulfatierungsmitteln, Phosphorylierungsmitteln, Halogenierungsmitteln, Alkyl- oder Arylsulfonsäurehalogeniden, Alkyl- oder Arylcarbonsäurehalogeniden oder Alkyl- oder Arylcarbonsäureanhydriden in die entsprechenden Produkte übergeführt werden, in denen die Gruppierung $-SO_2-Z$ z.B. die Gruppierung $-SO_2-CH_2-CH_2-O-SO_3H$, $-SO_2-CH_2-CH_2-O-PO_3H_2$, $-SO_2-CH_2-CH_2$-Halogen, $-SO_2-CH_2-CH_2-O-CO-CH_3$ oder $-SO_2-CH_2-CH_2-O-CO-C_6H_5$ bedeutet. Die so erhaltenen Produkte können ihrerseits durch Behandeln mit alkalisch wirkenden Mitteln, wie beispielsweise Alkalihydroxid oder Alkalicarbonat, wie Natriumhydroxid oder Natriumcarbonat, in entsprechende Verbindungen übergeführt werden, in denen die Gruppierung $-SO_2-Z$ die Gruppierung $-SO_2-CH=CH_2$ bedeutet. Die so erhaltenen Produkte können wiederum durch Umsetzung (Addition) mit Salzen der Thioschwefelsäure, wie Natriumthiosulfat, in Verbindungen übergeführt werden, in denen die Gruppierung $-SO_2-Z$ die Gruppierung $-SO_2-CH_2-CH_2-S-SO_3H$ bedeutet.

Geeignete Sulfatierungsmittel sind hierbei beispielsweise konzentrierte Schwefelsäure sowie Chlorsulfonsäure und Amidosulfonsäure oder andere Schwefeltrioxid abgebende Verbindungen. Geeignete Phosphorylierungsmittel sind hierbei beispielsweise konzentrierte Phosphorsäure, Pyro-, Meta- oder Polyphosphorsäurealkylester, Phosphoroxichlorid oder Gemische aus Phosphorsäure und Phosphor-(V)-oxid. Als Halogenierungsmittel können beispielsweise Thionylchlorid oder Thionylbromid verwendet werden.

Die Sulfatierung der Hydroxygruppe in einer Verbindung der Formel (1) erfolgt z.B. durch Umsetzung mit konzentrierter Schwefelsäure bei 0 °C bis mässig erhöhter Temperatur. Die Sulfatierung kann auch durch Reaktion der Hydroxyverbindung mit zwei Äquivalenten Chlorsulfonsäure pro Hydroxygruppe in einem polaren organischen Lösungsmittel, wie beispielsweise N-Methylpyrrolidon, bei 10 bis 80 °C erfolgen. Vorzugsweise erfolgt die Sulfatierung durch Eintragen der betreffenden Verbindung in Schwefelsäuremonohydrat bei Temperaturen zwischen 5 und 15 °C. Die Einführung eines anderen Restes für Z in eine Verbindung der Formel (1) anstelle eines Halogenatoms oder der Sulfatogruppe, beispielsweise einer Thiosulfato- oder Phosphatogruppe, erfolgt in an sich bekannter Weise.

Die Verbindungen der Formel (2) oder (2a) können in an sich bekannter Weise hergestellt werden, z.B. indem man eine Verbindung der Formel

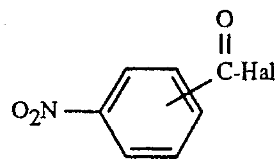

(6)

worin Hal Halogen, insbesondere Chlor, bedeutet, mit einem Amin der Formel

$NH-(alk)-CH_2-SO_2-Z$
|
V

(3a₁)

T
|
$NH-(alk)-CH_2-SO_2-Z$
|
R'

(3b₁)

$NH-(CH_2)_p-O-(CH_2)_q-SO_2-Z$
|
R'

(3c₁)

$NH-(CH_2)_p-NH-(CH_2)_q-SO_2-Z$
|
R'

(3d₁)

$NH-(CH_2)-_p-N[(CH_2)_q-SO_2-Z]_2$
|
R'

(3e₁) oder

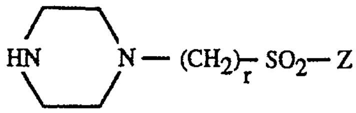

(3f1),

worin (alk), V, T, R′, p, q, r und Z jeweils die zuvor angegebene Bedeutung haben, umsetzt, und gegebenenfalls den Rest Z in einen anderen Rest Z verwandelt.

Die Nitroverbindungen der Formel (6) sind an sich bekannt oder können nach bekannten Methoden erhalten werden.

Die Umsetzung (Kondensation) des Säurechlorids der Formel (6) mit den vorgenannten Aminen erfolgt zum Beispiel in wässrigem, wässrig-organischem oder organischem Medium bei einer Temperatur zwischen 0° und 120° C, vorzugsweise 100° bis 60° C, in Gegenwart von alkalischen säurebindenden Mitteln, z.B. Alkalimetallhydroxiden, -carbonaten oder -bicarbonaten.

Als Beispiele für Verbindungen der Formel (2) seien genannt:
3-Nitrobenzoesäure-N-[β-(β′-hydroxyäthylsulfonyl)-äthyl]-amid, 4-Nitrobenzoesäure-N-[β-(β′-hydroxyäthylsulfonyl)-äthyl]-amid, 3-Nitrobenzoesäure-N-[ß-(ß′-chloräthylsulfonyl)-äthyl]-amid, 4-Nitrobenzoesäure-N-[β-(β′-chloräthylsulfonyl)-äthyl]-amid, 3-Nitrobenzoesäure-N[β-(β′-sulfatoäthylsulfonyl)-äthyl]-amid, 3-Nitrobenzoesäure-N-β-(vinylsulfonyl)-äthylamid, 4-Nitro-benzoesäure-N-β-(vinylsulfonyl)-äthylamid.

Das erfindungsgemässe Verfahren weist gegenüber den bekannten Verfahren zur Herstellung der Azoverbindung der Formel (1) folgende Vorteile auf:

Durch die selektive Reduktion bzw. Hydrierung der aromatischen Nitroverbindung der Formel (2) in Gegenwart einer Mono- oder Dicarbonsäure und in Gegenwart von Nickelkatalysatoren kann die Umsetzung quantitativer erfolgen als es bisher möglich war.

Durch die höhere Reinheit der gemäss dem erfindungsgemässen Verfahren erhaltenen Verbindungen verglichen mit den auf übliche Weise hergestellt gleichen Verbindungen können Reaktivfarbstoffe mit reproduzierbar verbesserten Eigenschaften erhalten werden.

Die nachfolgenden Beispiele dienen der Veranschaulichung der Erfindung. Darin sind die Teile Gewichtsteile und die Prozente Gewichtsprozente. Die Temperaturen sind in Celsiusgraden angegeben. Die Beziehung zwischen Gewichtsteilen und Volumenteilen ist dieselbe wie diejenige zwischen Gramm und Kubikzentimeter.

Beispiel 1:

306,6 Teile 3-Nitrobenzoesäure-N-β-(β′-hydroxyäthylsulfonyl)-äthylamid, 15 Teile wasserfeuchtes Raney-Nickel (entsprechend 9,2 Teile trockenem Raney-Nickel), 2,4 Teile 80%-iger Essigsäure und 400 Teile Wasser werden in einem Autoklaven mit Begasungsrührer eingetragen und Wasserstoff aufgepresst. Bei einem Druck von 10 bar und einer Temperatur von 80° C wird hydriert. Die Hydrierzeit beträgt 1 1/4 Stunden. Man erhält in quantitativer Ausbeute 3-Aminobenzoesäure-N-β-(β′-hydroxyäthylsulfonyl)-äthylamid mit einer Reinheit von >99 % (flüssigchromatographische Analyse).

Beispiel 2:

230 Teile 3-Nitrobenzoesäure-N-β-(β′-hydroxyäthylsulfonyl)-äthylamid, 3,45 Teile eines mit Magnesiumoxid aktivierten Nickel-Kieselgur-Trägerkatalysators (55 Gewichts-% Ni), 0,34 Teile Essigsäure und 100 Teile Wasser werden in einen Autoklaven mit Begasungsrührer eingetragen und Wasserstoff aufgepresst. Bei einem Druck von 20 bar und einer Temperatur von 120° C wird hydriert. Die Hydrierzeit beträgt 1,5 Stunden. Man erhält mit einer Ausbeute von 98% d. Th. 3-Aminobenzoesäure-N-β-(β′-hydroxyäthylsulfonyl)-äthylamid.

Verwendet man anstelle des mit Magnesiumoxid aktivierten Nickel-Kieselgur-Trägerkatalysators einen mit Zirkonium aktivierten Nickel-Kieselgur-Trägerkatalysator (mit 60 Gewichtsprozent Nickel), werden gleichgute Resultate erhalten.

Verwendet man anstelle der 230 Teile 3-Nitrobenzoesäure-N-β-(β′-hydroxyäthylsulfonyl)-äthylamid, 230 Teile 4-Nitrobenzoesäure-N-β-(β′-hydroxyäthylsulfonyl)-äthylamid so erhält man mit guter Ausbeute 4-Aminobenzoesäure-N-β-(β′-hydroxyäthylsulfonyl)-äthylamid.

Beispiel 3:

306,6 Teile 4-Nitrobenzoesäure-N-β-(β-hydroxyäthylsulfonyl)-äthylamid), 15 Teile wasserfeuchtes Raney-Nickel (entsprechend 9,2 Teile trockenem Raney-Nickel), 2,4 Teile Essigsäure und 400 Teile Wasser werden in einem Autoklaven mit Begasungsrührer eingetragen und Wasserstoff aufgepresst. Bei einem Druck von 10 bar und einer Temperatur von 80° C wird hydriert. Die Hydrierzeit beträgt 1 1/4 Stunden. Man erhält in quantitativer Ausbeute 4-Aminobenzoesäure-N-β-(β′-hydroxyäthylsulfonyl)-äthylamid.

Beispiel 4:

230 Teile 3-Nitrobenzoesäure-N-β-(β′-hydroxyäthylsulfönyl)-äthylamid, 3,45 Teile eines mit Magnesiumoxid aktivierten Nickel-Kieselgur-Trägerkatalysator (55 Gewichts-% Ni), 0,34 Teile Essigsäure und 100 Teile Wasser werden in einen Autoklaven mit Begasungsrührer eingetragen und Wasserstoff aufgepresst. Bei einem Druck von 5 bar und einer Temperatur von 80° C wird hydriert. Die Hydrierzeit beträgt 4,5 Stunden. Man erhält mit einer Ausbeute von 98% d. Th. 3-Aminobenzoesäure-N-β-(β′-hydroxyäthylsulfonyl)-äthylamid.

Beispiel 5:

306,6 Teile 3-Nitrobenzoesäure-N-β-(β′-hydroxyäthylsulfönyl)-äthylamid), 15 Teile wasserfeuchtes Raney-Nickel (entsprechend 9,2 Teile trockenem Raney-Nickel), 3,75 Teile Essigsäure und 400 Teile Wasser werden in einem Autoklaven mit Begasungsrührer eingetragen und Wasserstoff aufgepresst. Bei einem Druck von 5 bar und einer Temperatur von 65° C wird hydriert. Die Hydrierzeit beträgt 3 Stunden. Man erhält in quantitativer Ausbeute 3-Aminobenzoesäure-N-β-(β′-hydroxyäthylsulfonyl)-äthylamid.

Wenn man wie in den Beispielen 1 bis 5 angegeben arbeitet und anstelle von Essigsäure eine äquimolare Menge Propionsäure, Malonsäure oder Benzoesäure verwendet, so werden ähnlich gute Ergebnisse erhalten.

**Ansprüche**

1. Verfahren zur Herstellung von aromatischen Aminen der Formel

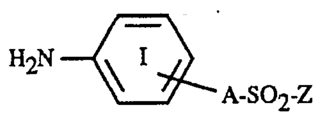

(1),

worin A ein Brückenglied, Z ein Rest der Formel $-CH_2-CH_2-OH$, $-CH=CH_2$ oder $-CH_2-CH_2-Y$, und Y eine unter alkalischen Bedingungen abspaltbare Gruppe ist, und der Benzolkern 1 weitersubstituiert sein kann, dadurch gekennzeichnet, dass man Verbindungen der Formel

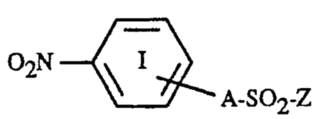

(2),

worin A, Z und I die unter Formel (1) angegebenen Bedeutungen haben, bei einem Druck von 0,1 bis 100 bar in Gegenwart einer niederen aliphatischen oder aromatischen Mono-oder Dicarbonsäure und in Gegenwart eines Nickel-Katalysators hydriert.

2. Verfahren gemäss Anspruch 1, dadurch gekennzeichnet, dass man bei einer Temperatur von 30 bis 150° C, insbesondere bei einer Temperatur zwischen 50 und 140° C hydriert.

3. Verfahren gemäss einem der Ansprüche 1 und 2, dadurch gekennzeichnet, dass man bei einem pH-Wert von 4,5 bis 7, insbesondere bei einem pH-Wert von 5 bis 7 hydriert.

4. Verfahren gemäss einem der Ansprüche 1 bis 3, dadurch gekennzeichnet, dass man in Gegenwart eines Nickel-Trägerkatalysators, oder Raney-Nickel hydriert.

5. Verfahren gemäss einem der Ansprüche 1 bis 4, dadurch gekennzeichnet, dass man bei einem Druck von 1 bis 40 bar hydriert.

6. Verfahren gemäss einem der Ansprüche 1 bis 5, dadurch gekennzeichnet, dass man in Gegenwart von 0,5 bis 20 Gewichtsprozent, insbesondere zwischen 1 bis 10 Gewichtsprozent an Nickel-Katalysator, bezogen auf die Menge der Verbindung der Formel (2), hydriert.

7. Verfahren gemäss einem der Ansprüche 1 bis 6, dadurch gekennzeichnet, dass man als niedere aliphatische oder aromatische Mono- oder Dicarbonsäure eine $C_2$-$C_4$-Mono- oder Dicarbonsäure oder Benzoesäure verwendet.

8. Verfahren gemäss Anspruch 7, dadurch gekennzeichnet, dass man Essigsäure verwendet.

9. Verfahren gemäss einem der Ansprüche 1 bis 8, dadurch gekennzeichnet, dass man 0,1 bis 10 Molprozent einer niederen aliphatischen oder aromatischen Mono- oder Dicarbonsäure bezogen auf die Molmenge der Verbindung der Formel (2), verwendet.

10. Verfahren gemäss Anspruch 9, dadurch gekennzeichnet, dass man 3 bis 5 Molprozent verwendet.

11. Verfahren gemäss einem der Ansprüche 1 bis 10, dadurch gekennzeichnet, dass man in wässriger Lösung oder wässrigorganischer Lösung hydriert.

12. Verfahren gemäss Anspruch 11, dadurch gekennzeichnet, dass man in wässriger Lösung hydriert.

13. Verfahren gemäss einem der Ansprüche 1 bis 12, dadurch gekennzeichnet, dass man Verbindungen der Formel

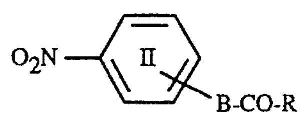

(2a)

verwendet worin B die direkte Bindung oder ein Rest der Formel

$$-(CH_2)_n- \text{ oder } -O-(CH_2)_n- ,$$

n = 1 bis 6, R ein Rest der Formel

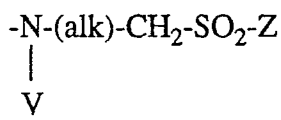

(3a),

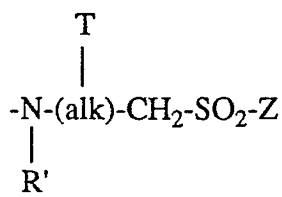

,

(3b),

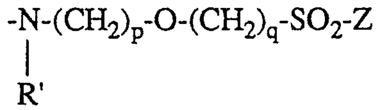

(3c),

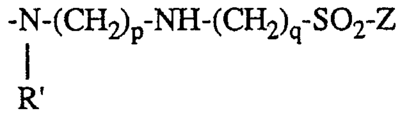

(3d),

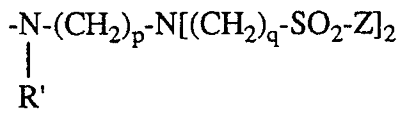

(3e) oder

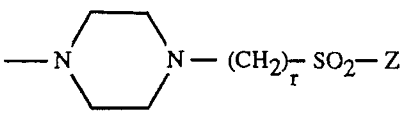

(3f),

worin R′ Wasserstoff oder $C_1$-$C_6$-Alkyl ist, (alk) einen $C_1$-$C_6$-Alkylenrest darstellt, T Wasserstoff, Halogen, Hydroxy, Sulfato, Carboxy, Cyano, $C_1$-$C_4$-Alkoxycarbonyl, Carbamoyl oder einen Rest $-SO_2$-Z bedeutet, V Wasserstoff, gegebenenfalls substituiertes $C_1$-$C_4$-Alkyl oder einen Rest der Formel -(alk)-$CH_2$-$SO_2$-Z, worin (alk) die zuvor angegebene Bedeutung hat, ist, Z die in Anspruch 1 angegebene Bedeutung hat, und p, q und r unabhängig voneinander eine ganze Zahl von 1 bis 6 bedeuten, ist, und der Benzolkern II weitere Substituenten enthalten kann.

14. Verfahren gemäss einem der Ansprüche 1 und 13, dadurch gekennzeichnet, dass man Verbindungen der Formel (2) oder (2a) verwendet, worin Z β-Hydroxyäthyl, β-Sulfatoäthyl, β-Thiosulfatoäthyl, β-Phosphatoäthyl, β-Acetoxyäthyl, β-Halogenäthyl oder Vinyl ist.

15. Verfahren gemäss einem der Ansprüche 1 bis 14, dadurch gekennzeichnet, dass der Benzolkern I bzw. II nicht weitersubstituiert ist.

16. Verfahren zur Herstellung von Verbindungen der Formel

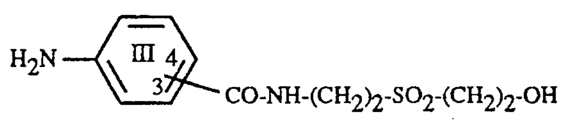

(4)

gemäss einem der Ansprüche 1 bis 5, wobei der Rest CO-NH-$(CH_2)_2$-$SO_2$-$(CH_2)_2$-OH in 3- oder 4-Stellung an den Benzring III gebunden ist, dadurch gekennzeichnet, dass man Verbindungen der Formel

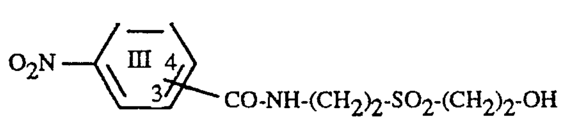

(5)

wobei der Rest CO-NH-$(CH_2)_2$-$SO_2$-$(CH_2)_2$-OH in 3- oder 4-Stellung an den Benzring III gebunden ist, bei einem Druck von 15 bis 25 bar, insbesondere 20 bar, bei einer Temperatur von 70 bis 90 °C, insbesondere 80 °C, in Gegenwart von 2 bis 4 Molprozent Raney-Nickel bezogen auf die Verbindung der Formel (5), insbesondere in Gegenwart von 3 Molprozent Raney-Nickel bezogen auf die Verbindung der Formel (5) bei einem pH-Wert von 5 bis 5,5 in Gegenwart von Essigsäure hydriert.

17. Die nach dem Verfahren gemäss Anspruch 1 erhaltenen Verbindungen der Formel (1).

18. Verwendung der gemäss dem Verfahren nach Anspruch 1 erhaltenen Verbindungen zur Herstellung von Reaktivfarbstoffen.

Europäisches Patentamt

# EUROPÄISCHER RECHERCHENBERICHT

Nummer der Anmeldung

EP 90810609.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| A | DE - A1 - 3 733 504 (HOECHST) * Ansprüche 5,8 * -- | 1 | C 07 C 317/28 |
| A | EP - A1 - 0 313 513 (CIBA-GEIGY) * Seite 5, Zeile 60; Seite 3, Zeilen 5-49; Seite 7, Zeilen 44-54 * -- | 1 | |
| A | DE - B - 2 240 849 (FRABWERKE HOECHST) * Anspruch 1 * ---- | 1,3,4, 7 | |

RECHERCHIERTE SACHGEBIETE (Int Cl 5)

C 07 C 317/00
C 07 C 315/00
C 09 B 62/00
C 07 C 209/00

Der vorliegende Recherchenbericht wurde für alle Patentansprüche erstellt

| Recherchenort | Abschlußdatum der Recherche | Prüfer |
|---|---|---|
| WIEN | 30-11-1990 | REIF |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82